# EUROPÄISCHE PATENTSCHRIFT

(11) **EP 3 802 671 B1**
(45) Veröffentlichungstag und Bekanntmachung des Hinweises auf die Patenterteilung: **25.06.2025**
(21) Anmeldenummer: 19726982.2
(22) Anmeldetag: 24.05.2019
(51) Int. Cl.: A61M 1/16, C08L 71/00, C08J 5/04, C08K 7/06, C08K 7/14, C08L 81/06

(54) **VERBUNDMATERIAL FÜR VERSCHLEISSARME MECHANISCHE BAUTEILE DER KRAFT- UND BEWEGUNGSÜBERTRAGUNG**
COMPOSITE MATERIAL FOR LOW-WEAR MECHANICAL COMPONENTS FOR FORCE AND MOTION TRANSMISSION
MATÉRIAU COMPOSITE POUR COMPOSANTS MÉCANIQUES À FAIBLE USURE POUR TRANSMISSION DE FORCE ET DE MOUVEMENT

(30) Priorität: 24.05.2018 DE 102018208149
(43) Veröffentlichungstag der Anmeldung: 14.04.2021
(73) Patentinhaber: Fresenius Medical Care Deutschland GmbH, 61352 Bad Homburg (DE)
(72) Erfinder: FISCHER, Gerome, 99947 Weberstedt (DE); PETERS, Arne, 61352 Bad Homburg (DE); KUNZ, Wolfgang, 97702 Münnerstadt (DE); COHEN, Scott, Newton, Massachusetts 02459 (US)
(74) Vertreter: Ricker, Mathias
(86) Internationale Anmeldenummer: PCT/EP2019/063538
(87) Internationale Veröffentlichungsnummer: WO 2019/224383

(56) Entgegenhaltungen:
- CN-B- 102 558 760
- JP-A- 2006 226 464
- US-A1- 2005 096 234
- US-B2- 7 575 800

## Beschreibung

### THEMA DER ERFINDUNG

Die Erfindung betrifft ein Verbundmaterial für mechanische Bauteile zur Kraft- und Bewegungsübertragung. Die Erfindung betrifft weiterhin ein mechanisches Bauteil für Kraft- und Bewegungsübertragungen, aufweisend das erfindungsgemäße Verbundmaterial. Die Erfindung betrifft weiterhin eine Zahnradpumpe aufweisend wenigstens ein Zahnrad, welches das erfindungsgemäße Verbundmaterial aufweist. Die Erfindung betrifft ebenfalls die Verwendung des erfindungsgemäßen Verbundmaterials für flüssigkeitsgelagerte mechanische Bauteile in medizinischen Pumpanwendungen.

### HINTERGRUND DER ERFINDUNG

Die vorliegende Erfindung betrifft das Gebiet der Verbundmaterialien. Verbundmaterialien werden für mechanische Bauteile zur Kraft- und Bewegungsübertragung verwendet. Insbesondere werden Verbundmaterialien aus Kunststoffen und Verstärkungsmaterialien verwendet, um den daraus hergestellten mechanischen Bauteilen eine höhere Festigkeit, eine höhere Verschleißfestigkeit, ein geringeres Gewicht und weitere verbesserte Eigenschaften zu verleihen. Kraft- und bewegungsübertragende mechanische Bauteile werden z.B. in Zahnradpumpen verwendet, insbesondere in Form von Zahnrädern.

Zahnradpumpen werden in der Medizintechnik, insbesondere in Dialysemaschinen, zur Förderung von therapeutischen Fluiden verwendet. Dabei handelt es sich um Pumpen zur Förderung von in der Dialysetherapie verwendeten Flüssigkeiten, oder um Entgasungspumpen zur Entgasung der in der Dialyse verwendeten Flüssigkeiten.

Eine Dialysemaschine weist einen Hydraulikkreis auf, der im Wesentlichen die Herstellung, Förderung und Bereitstellung von Dialyseflüssigkeit gewährleistet. In bestimmten Anwendungen wird dabei die Dialyseflüssigkeit in der Dialysemaschine aus bereitgestellten Dialysekonzentraten und Reinstwasser hergestellt. Das Reinstwasser wird dabei in einer Mischkammer mit den Dialysekonzentraten vermischt und zur gebrauchsfertigen Dialyseflüssigkeit umgesetzt. Für die Herstellung der Dialyseflüssigkeit ist es dabei unter anderem notwendig, das Reinstwasser zu entgasen und zu fördern. In der Regel wird dieser Prozesse mit dafür vorgesehenen Zahnradpumpen durchgeführt. Darüber hinaus weist die Dialysemaschine auch Pumpvorrichtungen auf, mit denen die Dialyseflüssigkeit zum Dialysefilter gefördert wird und für die extrakorporale Blutreinigung bereitgestellt wird. In der extrakorporalen Blutreinigung wird dabei das vom Patienten entnommene Blut mit Hilfe von Blutpumpvorrichtungen über eine Schlauchleitung zum Dialysefilter gefördert und dort über eine semipermeable Membran mit der ebenfalls zum Dialysefilter geförderten Dialyseflüssigkeit in Stoffaustausch gebracht und so eine Blutreinigung bewirkt. Nach Beendigung einer solchen Dialysebehandlung ist es aus hygienischer Sicht notwendig, den Hydraulikkreis der Dialysemaschine zu desinfizieren. Dabei wird ein Spülvorgang des Hydraulikkreises mit Reinstwasser und Desinfektionsmittel bei erhöhten Temperaturen von ca. 85°C durchgeführt. Als Desinfektionsmittel werden insbesondere oxidierende Mittel verwendet, um den Hydraulikkreis zu dekontaminieren.

Die im Hydraulikkreislauf einer Dialysemaschine verwendeten Zahnradpumpen sind demnach dafür vorgesehen, Dialyseflüssigkeiten, Reinstwasser, oxidierende Desinfektionsmittel sowie Flüssigkeits-Luft-Gemische bzw. auch nur Luft zur Entgasung fördern zu können. Die größte Pumpaktivität dieser Zahnradpumpen wird insbesondere für die Förderung von Dialyseflüssigkeiten verwendet. Zudem ist vorgesehen, mit derartigen Zahnradpumpen auch Pumpvorgänge durchzuführen, die unter erhöhten Temperaturen stattfinden, z.B. während einer Heißdesinfektion der Dialysemaschine, z.B. bei 85°C oder höher, z.B. bei bis zu 90°C oder 95°C.

Die in der Herstellung von Dialyseflüssigkeiten bekannten und verwendeten Zahnradpumpen sind derart konstruiert, dass die Zahnräder während des Pumpbetriebs in der zu fördernden Flüssigkeit schwimmend gelagert sind. Die Zahnräder sind dabei beispielsweise in einem Edelstahlgehäuse gelagert und können über eine drehende Magnetkopplung angetrieben werden. Üblicherweise werden für Zahnradpumpen in Dialysemaschinen Zahnräder aus einem Verbundmaterial verwendet, die aus einem polymeren Trägermaterial sowie einem Verstärkungsmaterial bestehen. Bekannt sind Zahnräder für Zahnradpumpen, die aus einem Verbundmaterial aus Polyetheretherketon (PEEK) und Carbonfasern bestehen. Die Verstärkung von PEEK mit Kohlenstofffaser bewirkt dabei eine höhere Festigkeit und eine geringere Wärmeausdehnung des Verbundmaterials im Vergleich zum reinen PEEK. Die Verstärkung des Trägermaterials mit dem Verstärkungsmaterial ist insbesondere bei Pumpen mit schwimmend gelagerten Zahnrädern notwendig, da hier die Zahnräder selbst die gesamte Lagerlast tragen und damit mechanisch stark belastet sind. Es bedarf folglich einer hohen mechanischen Festigkeit des Verbundmaterials, um dauerhaft den gewünschten Druck in dem zu pumpenden Fluid aufbauen zu können.

Aus Gründen der Hygiene wird für die Herstellung von Dialyseflüssigkeiten Reinstwasser mit einem möglichst hohen Reinheitsgrad verwendet. Für die Herstellung von Dialyseflüssigkeit wird vorzugsweise Reinstwasser verwendet, das eine elektrische Leitfähigkeit von 10⁻⁴ S/m und weniger aufweist. Eine geringe elektrische Leitfähigkeit wird dabei als Maß für eine hohe Reinheit des Reinstwassers angesehen. Die hohe Reinheit des Reinstwassers führt in Hinblick auf Zahnradpumpen, die die bekannten Verbundmaterialien aufweisen, jedoch zu einem nachteiligen Effekt. Es hat sich gezeigt, dass Verbundmaterialien, die Verstärkungsmaterialien mit einer hohen elektrischen Leitfähigkeit enthalten, wie z.B. Carbonfasern, in Kontakt mit Reinstwasser aufgrund der geringen elektrischen Leitfähigkeit des Reinstwassers einen hohen Verschleiß aufweisen. Die Standfestigkeit der aus diesen Verbundmaterialien hergestellten Zahnräder wird im Betrieb dadurch signifikant verkürzt. Insbesondere ist damit auch mit einer Kontamination der Fluide durch abgeriebene Partikel zu rechnen, was zu ernsthaften Problemen während der Dialysetherapie oder während des Heißreinigungsvorgangs führen kann.

Es sind Verstärkungsmaterialien mit geringer elektrischer Leitfähigkeit basierend auf anorganischen Materialien bekannt. Diese anorganischen Materialien weisen jedoch eine vergleichsweise hohe Wärmeausdehnung auf. Eine hohe Wärmeausdehnung führt bei mechanischen Bauteilen der Kraft- und Bewegungsübertragung ebenfalls zu erhöhtem Verschleiß. Entsprechende Verbundmaterialien aus einem polymeren Trägermaterial und einem anorganischen Verstärkungsmaterial sind daher für Anwendungen bei erhöhten Temperaturen ebenfalls ungeeignet. Insbesondere würden Zahnräder in Zahnradpumpen von Dialysemaschinen aus einem derartigen Verbundmaterial den Heißreinigungsbetrieb nicht ohne hohen Materialverschleiß überstehen.

Die WO 2015/019047 A1 offenbart Bauteile aus einem ersten Teil und einem zweiten Teil, wobei der erste Teil ein erstes semikristallines Polymer mit Phenylen-, Carbonyl- und Ethereinheiten umfasst. Der zweite Teil umfasst ein zweites semikristallines Polymer, das ebenfalls Phenylen-, Carbonyl- und Ethereinheiten umfasst. Das zweite Polymer hat dabei eine Schmelztemperatur, die niedriger ist als die Schmelztemperatur des ersten Polymers. In einer Ausführung können erstes Polymer und zweites Polymer Teil einer ersten und zweiten Zusammensetzung sein, die einen Füllstoff aufweisen, z.B. entweder Glasfaser oder Carbonfaser. In einer weiteren Ausführung beschreibt die WO 2015/019047 A1 ein Zahnrad, das aus zwei Teilen besteht, wobei der zweite Teil einen Träger definiert und der erste Teil die Zähne des Zahnrads umfasst. Der zweite Teil ist dabei durch ein faserförmiges Material, z.B. in Form eines Gewebes, verstärkt.

US 4,837,251 beschreibt eine Zusammensetzung für einen druckgeformten Kern einer Verbundstruktur. Die Zusammensetzung enthält ein thermoplastisches Harz, ausgewählt aus der Gruppe der Polyetheretherketone, Polyetherketone, Polyarylsulfide, Polyarylketone, Polyarylsulfon, oder Polyarylethersulfone. In einer Ausführung weist die Zusammensetzung Carbonfasern, Glasfasern und Glasmikrokugeln auf.

Die WO 02/10320 offenbart Kunststoffzusammensetzungen für die Herstellung von Kunststofflagern. In einer Ausführung besteht die Kunststoffzusammensetzung aus einem polymeren Matrixmaterial, ausgewählt aus der Gruppe umfassend Polyamidimid, Polyetherimid, Polyimid, Polyetheretherketon, Polyphenylensulfid, einem Flüssigkristallpolymer oder Mischungen davon. Weiterhin enthält die Kunststoffzusammensetzung gemäß dieser Ausführung Carbonfasern und ein Additiv, ausgewählt aus der Gruppe umfassend Bornitrid, Kohlenstoff, Graphit, Molybdensulfid, Talk, Tetrafluorethylen und Kombinationen davon.

Die im Stand der Technik offenbarten Verbundmaterialien zeigen keine zufriedenstellende Lösung für die formulierten Probleme. Insbesondere werden im Stand der Technik keine Verbundmaterialien offenbart, die eine geringe Wärmeausdehnung und dabei günstige Verschleißeigenschaften aufweisen, so dass sie sich für mechanische Bauteile der Kraft- und Bewegungsübertragung eignen.

### AUFGABE DER ERFINDUNG

In einem ersten Aspekt der Erfindung bestand daher die Aufgabe, ein Verbundmaterial für mechanische Bauteile der Kraft- und Bewegungsübertragung bereitzustellen, welches die zuvor genannten Nachteile des hohen Verschleißes bei Reinstwasserkontakt und den hohen Verschleiß, hervorgerufen durch Wärmemausdehnung, überwindet.

In einem weiteren Aspekt der Erfindung bestand die Aufgabe ein mechanisches Bauteil für die Kraft- und Bewegungsübertragung, insbesondere ein Zahnrad, bereitzustellen, bei dem die Verschleißeigenschaften bei Reinstwasserkontakt und bei Anwendungen bei erhöhten Temperaturen verbessert sind.

In einem weiteren Aspekt der Erfindung bestand die Aufgabe eine Zahnradpumpe bereitzustellen, die bei Pumpanwendungen mit Reinstwasser und bei erhöhten Temperaturen verbesserte Verschleißeigenschaften aufweist und damit längere Lebensdauer und eine geringere Fehleranfälligkeit aufweist.

### ZUSAMMENFASSUNG DER ERFINDUNG

In einem ersten Aspekt der Erfindung wird die Aufgabe durch ein Verbundmaterial für mechanische Bauteile der Kraft- und Bewegungsübertragung nach Anspruch 1 gelöst. Die Ansprüche 2 bis 12 stellen bevorzugte Ausführungsformen da.

In einem zweiten Aspekt der Erfindung wird die Aufgabe durch ein mechanisches Bauteil für die Kraft und Bewegungsübertragung nach Anspruch 13 gelöst.

In einem dritten Aspekt der Erfindung wird die Aufgabe durch eine Zahnradpumpe nach Anspruch 14 gelöst.

In einem vierten Aspekt der Erfindung wird die Aufgabe durch die Verwendung eines Verbundmaterials für flüssigkeitsgelagerte mechanische Bauteile in medizinischen Pumpanwendungen nach Anspruch 15 gelöst.
Die Erfindung ist demnach in den beigefügten Ansprüchen 1 bis 15 dargelegt.
CN 102 558 760 B offenbart ein Verbundmaterial bestehend aus 10-30 Gew.% Polyetheretherketon, 10-15 Gew.% PTFE, 4-14 Gew.% Polyethersulfon, 8-30 Gew.% Polyphenylsulfide, 5-10 Gew.% Kohlenstofffasern und 15-18 Gew. % Glasfasern.
JP 2006 226464 A offenbart ein Verbundmaterial aufweisend (A) Polyetheretherketon, (B) Glasfasern oder Carbonfasern und (C) Polytetrafluoroethylen.
US 7 575 800 B2 offenbart ein Kompositmaterial mit einem faserförmigen Füllstoff und einer Matrix-Komponente, die Polyetheretherketon sein kann, wobei der faserförmige Füllstoff aus Carbonfaser besteht.

### DETAILLIERTE BESCHREIBUNG DER ERFINDUNG

In einem ersten Aspekt betrifft die Erfindung ein Verbundmaterial für mechanische Bauteile zur Kraft- und Bewegungsübertragung enthaltend oder bestehend aus zumindest drei Komponenten
(i) mindestens 45 Gew.% bezogen auf das Gesamtgewicht des Verbundmaterials eines Trägermaterials bestehend aus Polyetheretherketone,
(ii) 3 bis 20 Gew.% bezogen auf das Gesamtgewicht des Verbundmaterials eines ersten faserförmigen Verstärkungsmaterials, und
(iii) 10 bis 45 Gew.% bezogen auf das Gesamtgewicht des Verbundmaterials eines zweiten Verstärkungsmaterials, wobei
das erste faserförmige Verstärkungsmaterial einen Wärmeausdehnungskoeffizient aufweist, der geringer ist als der des zweiten Verstärkungsmaterials und wobei das zweite Verstärkungsmaterial eine elektrische Leitfähigkeit aufweist, die geringer ist als die des einen ersten Verstärkungsmaterials, und wobei sich die Gewichtsanteile der im Verbundmaterial enthaltenen Komponenten auf 100% addieren und wobei das erste faserförmige Verstärkungsmaterial Carbonfaser enthält oder aus Carbonfaser besteht und das zweite Verstärkungsmaterial Glasfaser enthält oder aus Glasfaser besteht.

Wie sich aus Obigem ergibt, kann das Verbundmaterial der vorliegenden Erfindung neben den genannten drei Komponenten weitere Komponenten enthalten, die je nach Anwendung und Bedarf frei gewählt werden können. In jedem Fall addieren sich die Anteile der vorhandenen Komponenten zu 100 Gew.-%.

In einer weiteren Ausführungsform gemäß des ersten Aspekts ist das Verbundmaterial dadurch gekennzeichnet, dass das erste faserförmige Verstärkungsmaterial eine Wärmeausdehnung von -0,15x10⁻⁶/K bis 2x10⁻⁶/K parallel zur Faserrichtung aufweist und das zweite Verstärkungsmaterial eine elektrische Leitfähigkeit von 10⁻⁴ S/m oder weniger aufweist.

Das Verbundmaterial zeichnet sich gegenüber den im Stand der Technik bekannten Verbundmaterialien, die nur ein Verstärkungsmaterial mit einer hohen elektrischen Leitfähigkeit und einer geringen thermischen Ausdehnung, wie z.B. Carbonfaser, aufweisen, durch einen deutlich verringerten Verschleiß aus. Zudem ist der Wärmeausdehnungskoeffizient des Verbundmaterials gering, so dass sich das Verbundmaterial auch zur Herstellung von mechanischen Bauteilen für die Kraft und Bewegungsübertragung bei erhöhten Temperaturen eignet. Weiterhin zeichnet sich das erfindungsgemäße Verbundmaterial durch einen niedrigen Reibungskoeffizienten aus. Weiterhin zeichnet sich das erfindungsgemäße Verbundmaterial gegenüber den im Stand der Technik bekannten Verbundmaterialien, die nur ein Verstärkungsmaterial mit einer niedrigen elektrischen Leitfähigkeit aber eine hohe thermische Wärmeausdehnung aufweisen, wie z.B. Glasfaser, durch eine deutlich verringerten Verschleiß aus. Weiterhin wurde festgestellt, dass das Verbundmaterial auch gegenüber oxidativen Einwirkungen eine hohe Beständigkeit aufweist.

Die Bestimmung der thermischen (Wärme)- Ausdehnung der Verstärkungsmaterialien erfolgt nach bekannten Methoden der Thermomechanischen Analyse (TMA) im technisch relevanten Temperaturbereich von 10 bis 110°C. Insbesondere ist die Norm DIN 51045-1:2005-8 zur Bestimmung der thermischen Längenänderung fester Körper bekannt. Die thermische Ausdehnung und elektrische Leitfähigkeit von Verstärkungsmaterialien ist zudem in der Fachliteratur dokumentiert.

Unter dem Begriff "*Verbundmaterial*" wird im Sinne der vorliegenden Anmeldung eine stoffschlüssige Masse von unterschiedlichen Materialien verstanden, die sich hinsichtlich ihrer Materialeigenschaften unterscheiden. Die Materialien unterscheiden sich dabei durch mechanische Eigenschaften, wie z.B. Zugfestigkeit, thermischen Eigenschaften, wie z.B. der Wärmeausdehnung, der Schmelztemperatur oder der Glastemperatur, oder chemischen Eigenschaften. Insbesondere wird im Sinne der vorliegenden Anmeldung unter dem Begriff "Verbundmaterial" eine Masse aus einem Trägermaterial und Verstärkungsmaterialien verstanden. Als "*Trägermaterial*" wird dabei ein Material verstanden, dass im Verbundmaterial eine kontinuierliche Phase darstellt. Gemäß der vorliegenden Erfindung wird als Trägermaterial ein Polymer verwendet. Bevorzugt sind im Sinne der vorliegenden Erfindung Polymere, die eine hohe Schmelztemperatur und/oder eine hohe Glasübergangstemperatur aufweisen, so dass sie auch bei erhöhten Temperaturen formstabil bleiben. Daneben zeichnen sich diese Polymere aber auch durch ein temperaturabhängiges Erweichungsverhalten aus, so dass diese Polymere oberhalb ihrer Schmelztemperatur in der Schmelze verarbeitet werden können.

Als "*Verstärkungsmaterial*" wird im Sinne der vorliegenden Anmeldung ein Material verstanden, das gegenüber dem Trägermaterial eine höhere Zugfestigkeit und/oder Verschleißfestigkeit aufweist. Insbesondere verleiht das Verstärkungsmaterial im Verbund mit dem Trägermaterial dem Verbundmaterial eine höhere Zugfestigkeit. Gemäß der vorliegenden Erfindung sind wenigstens zwei Verstärkungsmaterialien vorgesehen. Verstärkungsmaterialien können faserförmig oder nicht faserförmig sein, z.B. partikelförmig oder plättchenförmig sein. Bekannte faserförmige Verstärkungsmaterialien sind z.B. Glasfaser, Carbonfaser, Asbestfaser, Silikafaser, Aluminiumoxidfaser, Zirkoniumoxidfaser, Bornitritfaser oder Silikonnitritfaser. Bekannte nicht faserförmige Verstärkungsmaterialien sind z.B. Glimmer, Silika, Talkum, Aluminiumoxid, Kaolin, Calciumsulfat, Calciumcarbonat, Titandioxid, Ferrit, Ton, Glaspulver, Zinkoxid, Eisenoxid, Quarzpulver, Magnesiumcarbonat, Graphit, Kohlenstoffpulver, z.B. auch in Form von Nanotubes. Gemäß der vorliegenden Erfindung enthält das erste Verstärkungsmaterial des Verbundmaterials Carbonfaser und das zweite Verstärkungsmaterial Glasfaser oder erstes und zweites Verstärkungsmaterialien bestehen daraus.

Das zumindest drei Komponenten enthaltende oder aus diesen bestehende Verbundmaterial gemäß der vorliegenden Anmeldung wird vorzugsweise in einem Compoundierprozess hergestellt. Dabei wird das polymere Trägermaterial mit dem ersten faserförmigen Verstärkungsmaterial und dem zweiten Verstärkungsmaterial bei einer erhöhten Temperatur gemischt, die vorzugsweise oberhalb der Schmelztemperatur des polymeren Trägermaterials liegt. Die erhöhte Temperatur liegt dabei unterhalb der Zersetzungstemperatur des polymeren Trägermaterials. Im Sinne der vorliegenden Anmeldung wird unter der "*Schmelztemperatur*" des polymeren Trägermaterials die nach Methoden der Dynamischen Differenz-Thermoanalyse (DSC) bestimmte Schmelztemperatur verstanden. Insbesondere ist für die Bestimmung der Schmelztemperatur von Kunststoffen die Norm DIN EN ISO 11357-3:2013-04 bekannt. Vorteilhafterweise kann das geschmolzene polymere Trägermaterial die Verstärkungsmaterialien leicht benetzen, so dass das erste faserförmige Verstärkungsmaterial und das zweite Verstärkungsmaterial gleichmäßig im polymeren Trägermaterial verteilt und umschlossen sind.

Das polymere Trägermaterial und das erste faserförmige Verstärkungsmaterial und das zweite Verstärkungsmaterial können konstant an einer Stelle des Compoundierprozesses zugeführt werden, wo sie gemischt, erhitzt und durch Extrusion zum Verbundmaterial geformt werden. In einem Beispiel kann eine Masse des faserförmigen Verstärkungsmaterials durch eine Schmelze des polymeren Trägermaterials geführt werden. Die Masse kann eine kontinuierliche Länge von faserförmigem Füllstoff oder, bevorzugter, eine Vielzahl von kontinuierlichen Filamenten umfassen, die zumindest in einem gewissen Ausmaß konsolidiert wurden. Die kontinuierliche faserige Masse kann z.B. ein Geflecht, Gewebe oder Vlies umfassen. Die Filamente, aus denen die faserige Masse besteht, können im Wesentlichen gleichmäßig oder stochastisch innerhalb der Masse verteilt sein und gegebenenfalls zusätzlich ohne Vorzugsrichtung ausgerichtet sein.

Alternativ kann das Verbundmaterial in einem Verfahren hergestellt werden, in dem eine vorbestimmte Menge des ersten polymeren Trägermaterials und eine vorbestimmte Menge des ersten faserförmigen Verstärkungsmaterials und des zweiten Verstärkungsmaterials durch Vermischen in der Schmelze erhalten werden.

Bevorzugt ist vorgesehen, dass durch die gewählte Herstellmethode das erste faserförmige Verstärkungsmaterial und das zweite Verstärkungsmaterial isotrop in dem Trägermaterial dispergiert werden. Alternativ kann die Verteilung des ersten Verstärkungsmaterials und des zweiten Verstärkungsmaterials in dem Trägermaterial nicht ortsabhängig sein und die Ausrichtung der Fasern keine Vorzugsrichtung aufweisen. Die Materialeigenschaften des Verbundmaterials sind so nicht von einer möglichen Ausrichtung des ersten faserförmigen Verstärkungsmaterials und des zweiten Verstärkungsmaterials in dem Trägermaterial abhängig. Die mechanischen Eigenschaften des Verbundmaterials und der aus dem Verbundmaterial hergestellten mechanischen Bauteile für die Kraft und Bewegungsübertragung sind daher in allen Ausrichtungen gleich, was einen Vorteil darstellt.

Das erste faserförmige Verstärkungsmaterial weist vorzugsweise einen Wärmeausdehnungskoeffizienten von -0,15x10⁻⁶/K bis 2x10⁻⁶/K parallel zur Faserrichtung auf. Der Wert des Wärmeausdehnungskoeffizienten des ersten, faserförmigen Verstärkungsmaterials ist gegenüber dem polymeren Trägermaterial sehr gering beziehungsweise negativ. Typische Wärmeausdehnungskoeffizienten von im Sinne der vorliegenden Erfindung geeigneten polymeren Trägermaterialien liegen zwischen 30x10⁻⁶/K und 60x10⁻⁶/K. Faserförmige Verstärkungsmaterialien, die einen negative Wärmeausdehnungskoeffizienten aufweisen, sind bevorzugt, da sie bis zu einem gewissen Grad der naturgemäß hohen thermischen Gesamtausdehnung des polymeren Trägermaterials entgegenwirken und für eine insgesamt geringe thermische Ausdehnung des Verbundmaterials verantwortlich sind. In bevorzugten Ausführungsformen weist das erste faserförmige Verstärkungsmaterial einen Wärmeausdehnungskoeffizienten von -0,12x10⁻⁶/K oder mehr, insbesondere -0,1x10⁻⁶/K oder mehr, insbesondere -0,8x10⁻⁶/K oder mehr, insbesondere 0,5x10⁻⁶/K oder mehr, insbesondere -0,02x10⁻⁶/K oder mehr, aber weniger als 1,8x10⁻⁶/K, insbesondere weniger als 1,5x10⁻⁶/K, insbesondere weniger als 1,2x10⁻⁶/K, insbesondere weniger als 1x10⁻⁶/K, insbesondere weniger als 0,8x10⁻⁶/K, insbesondere weniger als 0,5x10⁻⁶/K auf.

Erfindungsgemäß weist das zweite Verstärkungsmaterial eine elektrische Leitfähigkeit auf, die 10⁻⁴ S/m oder weniger beträgt. Die geringe Leitfähigkeit des zweiten Verstärkungsmaterials bewirkt eine verringerte Verschleißanfälligkeit des Verbundmaterials. Überraschenderweise wurde festgestellt, dass die spezifische Verschleißrate des erfindungsgemäßen Verbundmaterials unerwartet niedriger ist als die des Trägermaterials alleine oder die des Verbundmaterials bestehend allein aus dem polymeren Trägermaterial und dem ersten faserförmigen Verstärkungsmaterial oder die des Verbundmaterials bestehend allein aus dem polymeren Trägermaterial und dem zweiten Verstärkungsmaterial. In bevorzugten Ausführungsformen beträgt die elektrische Leitfähigkeit des zweiten Verstärkungsmaterials 10⁻⁵ S/m oder weniger, insbesondere 10⁻⁶ S/m oder weniger, insbesondere 10⁻⁷ S/m oder weniger, insbesondere 10⁻⁸ oder weniger, insbesondere 10⁻⁹ S/m oder weniger, insbesondere 10⁻¹⁰ S/m, oder weniger, mindestens aber mehr als 10⁻¹⁵ S/m, insbesondere mehr als 10⁻¹⁴ S/m, insbesondere mehr als, 10⁻¹³ S/m, insbesondere mehr als 10⁻¹² S/m, insbesondere mehr als 10⁻¹¹ S/m.

Faserförmige Verstärkungsmaterialien sind bevorzugt, da sie dem Trägermaterial eine höhere Festigkeit, insbesondere eine höhere Zugfestigkeit und eine höhere Kerbschlagzähigkeit verleihen. Je nach geforderter Festigkeit des Verbundmaterials und der vorgesehenen Beanspruchung als mechanisches Bauteil in der Kraft- und Bewegungsübertragung kann der Einsatz des zweiten Verstärkungsmaterials in Faserform dabei vorteilhaft sind.

In einer weiteren Ausführungsform gemäß dem ersten Aspekt ist die Erfindung dadurch gekennzeichnet, dass das polymere Trägermaterial eine Glasübergangstemperatur von 120 bis 200°C, bevorzugt von 125°C bis 190°C, weiter bevorzugt von 130°C bis 160°C aufweist. Die Bestimmung der Glasübergangstemperaturen kann z.B. nach Methoden der Dynamischen Differenz-Thermoanalyse (DSC) erfolgen. Insbesondere ist die Norm DIN EN ISO 11357-2:2014-07 zur Bestimmung der Glasübergangstemperatur bekannt. Oberhalb der Glasübergangstemperatur nimmt die thermisch bedingte Erweichung des polymeren Trägermaterials, insbesondere unter mechanischer Beanspruchung, mit steigender Temperatur zu. Die Glasübergangstemperatur ist daher als ein Maß für das thermische Erweichungsverhalten von Polymeren anzusehen. Insbesondere sind im Sinne der vorliegenden Erfindung Polymere mit einer Glastemperatur von 120°C und darüber als hart anzusehen und eignen sich daher bevorzugt als polymere Trägermaterialien für ein Verbundmaterial gemäß der vorliegenden Erfindung.

In einer weiteren Ausführungsform gemäß des ersten Aspekts ist die Erfindung dadurch charakterisiert, dass das polymere Trägermaterial einen Reibungskoeffizienten von 0,13 bis 0,21 µs und/ oder eine spezifische Verschleißrate von 2 bis 6x10⁻⁶ mm³/Nm gemessen nach dem "Block on the Ring Test" aufweist. Der für die Untersuchung verwendete "Block on the Ring Test" basiert auf der Beschreibung der Norm ASTM G77 - 17. Das zu untersuchende Werkstück wird dabei unter Last auf einem Metallring aufgesetzt und der Ring in Rotation versetzt. Der Reibungskoeffizient und die spezifische Verschleißrate werden gemäß dieser Versuchsanordnung bestimmt. Die Verwendung eines polymeren Trägermaterials mit einem Reibungskoeffizienten von 0,13 bis 0,21 µs und/oder eine spezifische Verschleißrate von 2 bis 6x10⁻⁶ mm³/Nm ist für die Herstellung des Verbundmaterials zusammen mit dem ersten faserförmigen Verstärkungsmaterial und dem zweiten Verstärkungsmaterial vorteilhaft, um insgesamt die vorteilhaften geringen Werte des Reibungskoeffizienten und der spezifischen Abnutzungsrate des Verbundmaterials einzustellen.

In einer weiteren Ausführungsform gemäß des ersten Aspekts ist die Erfindung dadurch gekennzeichnet, dass das polymere Trägermaterial aus Polyetheretherketon (PEEK) besteht. Dieses Polymer eignen sich hinsichtlich seiner Temperaturbeständigkeit, der mechanischen Eigenschaften, der chemischen Inertheit und hinsichtlich der Verarbeitbarkeit in der Schmelze besonders für die Herstellung des erfindungsgemäßen Verbundmaterials.

In einer weiteren Ausführungsform gemäß dem ersten Aspekt ist die Erfindung dadurch gekennzeichnet, dass das erste faserförmige Verstärkungsmaterial Carbonfasern enthält oder aus Carbonfasern besteht. Im Sinne der vorliegenden Anmeldung wird unter dem Begriff "*Carbonfaser*" eine Faser verstanden, die aus einer Kunststofffaser durch einen Pyrolyseprozess erhalten wird. Carbonfasern zeichnen sich durch eine hohe Zugfestigkeit und durch einen geringen, insbesondere negativen Wärmeausdehnungskoeffizienten parallel zur Faserrichtung aus. Bei einer isotropen Anordnung der Carbonfasern im erfindungsgemäßen Verbundmaterial kann damit die temperaturabhängige Ausdehnung des Verbundmaterials gering gehalten werden und damit der Verschleiß von mechanischen Bauteilen in der Kraft- und Bewegungsübertragung, die aus dem Verbundmaterial hergestellt sind, gering gehalten werden.

In einer weiteren Ausführung gemäß dem ersten Aspekt ist die Erfindung dadurch gekennzeichnet, dass das zweite Verstärkungsmaterial Glasfasern enthält oder aus Glasfasern besteht. Glasfasern zeichnen sich durch eine hohe Zugfestigkeit aus und verleihen dem Verbundmaterial dadurch eine erhöhte Bruchdehnung. Weiterhin zeichnet sich Glas durch eine geringe elektrische Leitfähigkeit aus. Der Verschleiß von mechanischen Bauteilen in der Kraft- und Bewegungsübertragung, die aus dem Verbundmaterial hergestellt sind und mit Reinstwasser in Kontakt kommen, ist dadurch verringert.

In einer weiteren Ausführungsform gemäß dem ersten Aspekt ist die Erfindung dadurch gekennzeichnet, dass der Anteil des ersten faserförmigen Verstärkungsmaterials in dem Verbundmaterial 3 Gew.% oder mehr, bevorzugt 4 Gew.% oder mehr, weiter bevorzugt 5 Gew.% oder mehr, weiter bevorzugt 6 Gew.% oder mehr, weiter bevorzugt 7 Gew.% oder mehr, und 20 Gew.% oder weniger, bevorzugt 19 Gew.% oder weniger, weiter bevorzugt 18 Gew.% oder weniger, weiter bevorzugt 17 Gew.% oder weniger, weiter bevorzugt 16 Gew.% oder weniger, weiter bevorzugt 15 Gew.% oder weniger, insbesondere 3 Gew.% bis 18 Gew.%, weiter bevorzugt 5 Gew.% bis 18 Gew.%, weiter bevorzugt 5 Gew.% bis 15 Gew.%, weiter bevorzugt 7% bis 15 Gew.% bezogen auf das Gesamtgewicht des Verbundmaterials beträgt. Ein zu hoher Anteil von mehr als 20 Gew.% des ersten faserförmigen Verstärkungsmaterials, Carbonfaser, bezogen auf das Gesamtgewicht des Verbundmaterials führt bei der Verwendung für mechanische Bauteile in der Kraft- und Bewegungsübertragung und in Kontakt mit Reinstwasser zu einem nicht akzeptablen hohen Verschleiß. Bei einem Anteil des ersten faserförmigen Verstärkungsmaterials, Carbonfaser, von weniger als 3 Gew.%, bezogen auf das Gesamtgewicht des Verbundmaterials, kann die hohe Wärmeausdehnung des polymeren Trägermaterials oder des zweiten Verstärkungsmaterials, Glasfaser, nicht mehr ausreichend kompensiert werden. Entsprechend kann bei erhöhten Temperaturen das mechanische Bauteil durch eine zu hohe Wärmeausdehnung in Anwendungen der Kraft- oder Bewegungsübertragung verschleißen.

In einer weiteren Ausführung gemäß dem ersten Aspekt ist die Erfindung dadurch gekennzeichnet, dass der Anteil des zweiten Verstärkungsmaterials, Glasfaser, in dem Verbundmaterial 10 Gew.% oder mehr, bevorzugt 12 Gew.% oder mehr, bevorzugt 14 Gew.% oder mehr, weiter bevorzugt 15 Gew.% oder mehr, weiter bevorzugt 17 Gew.% oder mehr und nicht mehr als 45 Gew.%, bevorzugt nicht mehr als 42 Gew.%, weiter bevorzugt nicht mehr als 40 Gew.%, weiter bevorzugt nicht mehr als 38 Gew.%, weiter bevorzugt nicht mehr als 35 Gew.%, weiter bevorzugt nicht mehr als 33 Gew% weiter bevorzugt nicht mehr als 30 Gew.%, insbesondere bevorzugt 10 Gew.% bis 40 Gew.%, weiter bevorzugt 15 Gew.% bis 35 Gew.%, weiter bevorzugt 15 Gew.% bis 30 Gew.%, bezogen auf das Gesamtgewicht des Verbundmaterials, beträgt.

Ein Anteil des zweiten Verstärkungsmaterials, Glasfaser, von mehr als 45 Gew.%, bezogen auf das Gesamtgewicht des Verbundmaterials, kann eine zu hohe Wärmeausdehnung des Verbundmaterials bewirken, was insbesondere bei mechanischen Bauteilen der Kraft- und Bewegungsübertragung, die aus dem erfindungsgemäßen Verbundmaterial hergestellt sind, bei erhöhten Temperaturen zu nicht akzeptablem Verschleiß führt. Ein zu geringer Anteil des zweiten Verstärkungsmaterials, Glasfaser, von weniger als 10 Gew.%, bezogen auf das Gesamtgewicht des Verbundmaterials, muss durch ein weiteres Verstärkungsmaterial, dem ersten faserförmigen Verstärkungsmaterial, kompensiert werden, damit das Verbundmaterial eine ausreichende Festigkeit für mechanische Bauteile der Kraft- und Bewegungsübertragung aufweist. Da diese Verstärkungsmaterialien wegen der höheren elektrischen Leitfähigkeit nicht inert gegen Reinstwasser sind, wäre bei der Verwendung solcher mechanischer Bauteile in Kontakt mit Reinstwasser mit einem nicht akzeptablen Verschleiß zu rechnen.

In einer weiteren Ausführung gemäß dem ersten Aspekt ist die Erfindung dadurch gekennzeichnet, dass der Anteil des polymeren Trägermaterials in dem Verbundmaterial 45 Gew.% oder mehr, bevorzugt 50 Gew.% oder mehr, weiter bevorzugt 55 Gew.% oder mehr, weiter bevorzugt 60 Gew.% oder mehr bezogen auf das Gesamtgewicht des Verbundmaterials, beträgt. Ein Anteil des polymeren Trägermaterials von weniger als 45 Gew.% kann dazu führen, dass das erste faserförmige Verstärkungsmaterial und das zweite Verstärkungsmaterial nicht vollständig vom polymeren Trägermaterial umschlossen sind. Die Festigkeit des Verbundmaterials kann dadurch herabgesetzt sein und es kann bei der Verwendung des Verbundmaterials für kraft- und bewegungsübertagende mechanische Bauteile zu einem erhöhten Verschleiß kommen.

In einer weiteren Ausführung gemäß dem ersten Aspekt ist die Erfindung dadurch gekennzeichnet, dass das Verbundmaterial ein Antioxidationsmittel aufweist. Das Antioxidationsmittel verhindert eine Degradation des Trägermaterials und des ersten faserförmigen Verstärkungsmaterials in dem Verbundmaterial, die durch Ionen- oder Radikalbildung hervorgerufen werden.

Insbesondere werden an Verbundmaterialen, die in Bauteilen für die Kraft und Bewegungsübertragung verwendet werden, durch Reibungskontakt und dem daraus folgendem triboelektrischen Effekt Ladungen induziert. Im Falle des gemäß dem ersten Aspekt definierten Verbundmaterials, werden die elektrischen Ladungen durch homolytische und heterolytische kovalente Bindungsspaltung in dem Trägermaterial und/ oder dem ersten faserförmigen Verstärkungsmaterial, also insbesondere dem Carbonfasermaterial, induziert.

Die heterogene Bindungsspaltung erzeugt dabei ein Ionenpaar durch Spaltung kovalenter Bindungen, wobei die ionischen Stellen an den molekularen Spaltfragmenten des Trägermaterials oder dem faserförmigen Verstärkungsmaterial lokalisiert sind. Die auf diese Weise im Trägermaterial oder im ersten faserförmigen Verstärkungsmaterial erzeugten ionischen Stellen werden im Sinne der vorliegenden Anmeldung als Mechano-Ionen bezeichnet, da sie durch mechanischen Reibungskontakt entstehen. Die Mechano-Ionen umfassen kationische Mechano-Ionen, also positiv geladene molekulare Spaltfragmente, und anionische Mechano-Ionen, d.h. negativ geladene molekulare Spaltfragmente.

Bei der homolytischen Bindungsspaltung entsteht ein Radikalpaar, wobei die radikalischen Stellen an den jeweiligen molekularen Spaltfragmenten lokalisiert sind. Derartige radikalische Stellen werden im Sinne der vorliegenden Anmeldung als Mechano-Radikale bezeichnet, da Sie durch mechanischen Reibungskontakt erzeugt werden.

Mechano-Ionen und Mechano-Radikale verursachen chemische Folgereaktionen, die ihrerseits neue kovalente Bindungsspaltungen hervorrufen und so zur Degradation und zum Verlust der Festigkeit des Verbundmaterials beitragen. Sofern die Bildung von derartigen Machano-Ionen und Machano-Radikalen entgegengewirkt wird kann einer Schwächung des Verbundmaterials vermieden werden. Reinstwasser mit einer Leitfähigkeit von 10⁻⁴ S/m ist dabei insbesondere nicht geeignet mit Mechano-Ionen und Mechano-Radikalen in ausreichendem Maß zu reagieren, da es nur wenige Ionen aufweist, die mit den Mechano-Ionen oder Mechano-Radikalen im Verbundmaterial abreagieren können. Es erweist sich daher als Problem, dass Verbundmaterialien gemäß dem ersten Aspekt der Erfindung in der Verwendung als kraft- oder bewegungsübertragendes Bauteil und in Verbindung mit Reinstwasser bei zu hoher Reibungsbeanspruchung durch den tribologischen Effekt an Festigkeit verlieren.

Die Festigkeit der Verbundmaterialien gemäß dem ersten Aspekt der Erfindung kann unter Bedingungen hoher Reibungsbeanspruchung und bei Reinstwasserkontakt durch einen Anteil von Antioxidationsmittel in dem Verbundmaterial weiter erhöht werden.

Die Herstellung eines Verbundmaterials, dass Antioxidationsmittel enthält, kann durch Schmelzextrusion erfolgen, indem die Komponenten des Trägermaterials, des ersten faserförmigen Verstärkungsmaterials, Carbonfaser, des zweiten Verstärkungsmaterials, Glasfaser, und Antioxidationsmittel zum Verbundmaterial kompoundiert werden. Im Sinne der vorliegenden Anmeldung ist unter dem Begriff "*Antioxidationsmittel*" ein Stoff oder mehrere Stoffe zu verstehen, die bezüglich Ionen oder Radikalen chemisch reaktiv sind. Insbesondere werden gemäß der vorliegenden Anmeldung solche Antioxidationsmittel verwendet, die gegenüber gebildeten Mechano-Ionen und Mechano-Radikalen im Verbundmaterial gemäß des ersten Aspekts der Erfindung reaktiv sind.

Insbesondere können als Antioxidationsmittel Verbindungen, wie Tocopherole, Tocotrienole, Resveratrole, Flavonoide, H-Donoren, wie z.B. aromatische Amine und sterisch gehinderte Phenole, Hydroperoxidzersetzer, wie z.B. Phosphite, Phosphonite, Thiosynergisten, Alkylradikalfängern, wie z.B. sterisch gehinderte Aminstabilisatoren, Hydroxylamine, Benzofuranone, Acryloyl-modifizierte Phenole, oder multifunktionelle Stabilisatoren der vorgenannten Art, oder Mischung von Stabilisatoren der vorgenannten Art verwendet werden.

In einer weiteren Ausführungsform gemäß dem ersten Aspekt ist die Erfindung dadurch gekennzeichnet, dass es sich bei dem Antioxidationsmittel um ein primäres und/ oder ein sekundäres Antioxidationsmittel handelt. Bei den primären Antioxidationsmitteln kann es sich um sterisch gehinderte Phenole oder sekundäre aromatische Amine handeln. Der Begriff "sterisch" bezeichnet dabei auf molekularer Ebene räumlich anspruchsvolle Molekülgruppen. Ein bevorzugtes primäres Antioxidationsmittel ist das kommerziell erhältliche Evernox 1330, das die Verbindung 3,3',3',5,5',5'-hexa-tert-butyl-a,a',a'-(Mesitylen-2,4,6trityl)tri-p-Cresol aufweist. Bei den sekundären Antioxidationsmitteln kann es sich um Peroxide, organische Hydroperoxide, Phophite, Thioether oder organische Sulfide handeln. Ein bevorzugtes sekundäres Antioxidationsmittel ist das kommerziell erhältliche Doverphos S-9228, das die Verbindung Bis (2,4-dicumylphenyl) pentaerythritol diphosphite aufweist. Die Verwendung von primären Antioxidationsmitteln ist bevorzugt.

In einer weiteren Ausführungsform gemäß dem ersten Aspekt ist die Erfindung dadurch gekennzeichnet, dass der Anteil des Antioxidationsmittels in dem Verbundmaterial 0,001 Gew.% bis 2,5 Gew.%, bevorzugt 0,01 Gew.% bis 2,0 Gew.%, weiter bevorzugt 0,1 Gew.% bis 1 Gew.% bezogen auf das Gesamtgewicht des Verbundmaterials, beträgt. Insbesondere wird durch den Anteil des Antioxidationsmittels im angegebenen Bereich die Materialeigenschaften des Verbundmaterials hinsichtlich elektrischer Leitfähigkeit und Wärmausdehnung nur unwesentlich beeinflusst, so dass ein Verschleiß des Verbundmaterials in der Verwendung als mechanisches Bauteil in der Kraft- und Bewegungsübertragung bei Reinstwasserkontakt und Wärmemausdehnung nicht zum Tragen kommt.

Weiter insbesondere kann das vorliegende Verbundmaterial gemäß dem ersten Aspekt der Erfindung dadurch gekennzeichnet sein, dass die Anteile des Trägermaterials, des ersten faserförmigen Verstärkungsmaterials und des zweitem Verstärkungsmaterials und des Antioxidationsmittels zusammen 100 Gew.% ergeben.

In einer weiteren Ausführungsform gemäß dem ersten Aspekt ist die Erfindung dadurch charakterisiert, dass das Gewichtsverhältnis des zweiten Verstärkungsmaterials zum faserförmigen ersten Verstärkungsmaterial in dem Verbundmaterial 3:1 bis 1:1 beträgt. Es hat sich gezeigt, dass die Verschleißrate des Verbundmaterials in der Verwendung von kraft- und bewegungsübertragenden mechanischen Bauteilen in diesem Bereich der Gewichtsverhältnisse die geringsten Werte aufweist.

In einer weiteren Ausführungsform gemäß dem ersten Aspekt ist die Erfindung dadurch gekennzeichnet, dass die Fasern des faserförmigen ersten Verstärkungsmaterials und/oder die Fasern des faserförmigen zweiten Verstärkungsmaterials einen Faserdurchmesser quer zur Längserstreckung der Fasern von 1 µm oder mehr, bevorzugt 2 µm oder mehr, weiter bevorzugt 3 µm oder mehr und 10 µm oder weniger, weiter bevorzugt 9 µm oder weniger, weiter bevorzugt 8 µm oder weniger, insbesondere 1 µm bis 10 µm, 2 µm bis 9 µm, weiter bevorzugt 3 bis 8 µm aufweisen.

In einer weiteren Ausführungsform gemäß dem ersten Aspekt ist die Erfindung dadurch gekennzeichnet, dass die Fasern des faserförmigen ersten Verstärkungsmaterials und/oder die Fasern des zweiten faserförmigen Verstärkungsmaterials eine Länge von 10 µm oder mehr, bevorzugt 15 µm oder mehr, bevorzugt 20 µm oder mehr, bevorzugt 25 µm oder mehr und 60 µm oder weniger, bevorzugt 55 µm oder weniger, weiter bevorzugt 50 µm oder weniger, insbesondere von 10 µm bis 60 µm, bevorzugt von 15 µm bis 55 µm, weiter bevorzugt von 20 µm bis 50 µm aufweisen. Bei Faserlängen oberhalb von 60 µm kann eine vorteilhaft isotrope Verteilung der Fasern im Verbundmaterial verhindert sein. Insbesondere bei kleinen mechanischen Bauteilen kann es bei einer Faserlänge oberhalb von 60 µm in der Herstellung der Bauteile zu einer Ausrichtung der Fasern kommen, die sich negativ auf die Festigkeit der Bauteile auswirken kann. Bei geringer Länge der Faser von 10 µm oder darunter kann der vernetzende Charakter des faserförmigen ersten oder zweiten Verstärkungsmaterials in dem Verbundmaterial verloren gehen und zu einer verringerten Festigkeit des Verbundmaterials führen.

Insbesondere ist ein bevorzugter Bereich von Durchmesser und Länge der Fasern des faserförmigen ersten und des faserförmigen zweiten Verstärkungsmaterials so gewählt, dass ein Verhältnis von Durchmesser zu Länge der Fasern des ersten faserförmigen Verstärkungsmaterials und der Fasern des zweiten faserförmigen Verstärkungsmaterials 1:2 bis 1:20 beträgt. Dabei weisen mindestens 60%, bevorzugt mindestens 70%, weiter bevorzugt mindestens 80% der Fasern des faserförmigen ersten und des faserförmigen zweiten Verstärkungsmaterials dieses bevorzugte Verhältnis auf.

Eine weitere Ausführungsform des ersten Aspekts der Erfindung ist dadurch gekennzeichnet, dass das Verbundmaterial eine weitere Komponente zur Reibmodifizierung aufweist. Bei der weiteren Komponente handelt es sich z.B. um Bornitrit und/oder Teflon. Der Anteil der weiteren Komponente zur Reibmodifizierung beträgt 15 Gew.% oder weniger, bevorzugt 10 Gew.% oder weniger, weiter bevorzugt 5% Gew.% oder weniger, mindestens, sofern die Komponente zur Reibmodifizierung in dem Verbundmaterial vorgesehen ist, 0,2 Gew.% oder mindestens 0,5 Gew.%, oder mindestens 1 Gew.% bezogen auf das Gesamtgewicht des Verbundmaterials.

In einer weiteren Ausführungsform gemäß dem ersten Aspekt ist die Erfindung dadurch charakterisiert, dass die Anteile des polymeren Trägermaterials, des ersten faserförmigen Verstärkungsmaterials und des zweiten Verstärkungsmaterials zusammen 97 Gew.% oder mehr, insbesondere 100 Gew.% ergeben.

In einem zweiten Aspekt betrifft die Erfindung ein mechanisches Bauteil für Kraft- und Bewegungsübertragungen, aufweisend ein Verbundmaterial nach wenigstens einer Ausführungsform gemäß dem ersten Aspekt der Erfindung. Das erfindungsgemäße mechanische Bauteil zeichnet sich durch einen geringen Verschleiß aus. Insbesondere weist das mechanische Bauteil auch in Kontakt mit Reinstwasser einen geringen Verschleiß auf.

In einer Ausführungsform des zweiten Aspekts betrifft die Erfindung ein mechanisches Bauteil, wobei es sich bei dem mechanischen Bauteil um ein Zahnrad handelt.

In einem dritten Aspekt betrifft die Erfindung eine Zahnradpumpe, die dadurch gekennzeichnet ist, dass die Zahnradpumpe wenigstens ein Zahnrad nach einer Ausführungsform gemäß des zweiten Aspekts der Erfindung aufweist. Bauartbedingt sind die Zahnräder der Zahnradpumpe in dem zu fördernden Fluid gelagert. Der Vorteil der erfindungsgemäßen Zahnradpumpe ist darin zu sehen, dass diese einen geringen Verschleiß gegenüber der Förderung von Reinstwasser, insbesondere auch unter erhöhten Temperaturen, aufweist. Weiter wurde festgestellt, dass die Zahnradpumpe auch bei Förderung von oxidierenden Desinfektionsmitteln, z.B. in der Heißreinigung von medizinischen Vorrichtungen, verschleißarm ist. Die erfindungsgemäße Zahnradpumpe ist daher für den Einsatz im medizintechnischen Bereich und insbesondere als Pumpvorrichtung in Dialysemaschinen geeignet.

### BEISPIELE

### Block on the Ring Test

Die Durchführung des "Block on the Ring Tests" erfolgte in Anlehnung an die Beschreibung der ASTM G77-17. Es wurde ein Probenkörper aus dem zu untersuchenden Verbundmaterial mit den Maßen 4x4x17mm hergestellt. Der Probenkörper wurde in einem Teststand aufgenommen und auf einen Ringkörper aufgesetzt. Der Ringkörper bestand aus einem CrNiMo Stahl. Im Anschluss wurde der Probenkörper mit einer Kraft von 2,5 MPa beaufschlagt und auf die Außenfläche des Rings gedrückt. Der Ring wurde in Drehung versetzt, sodass der Probenkörper mit einer relativen Geschwindigkeit von 0,5 m/s über den Kontaktbereich des Ringkörpers gleitet. Die Testtemperatur von Ring- und Probenkörper wurde auf 23°C eingestellt. Probenkörper und Ring wurden während des Testverlaufs mit Wasser umspült. Es wurde eine relative Gesamtgleitstrecke von 36000 m durchlaufen.

### (1) Spezifische Verschleißrate

Zur Ermittlung der spezifischen Verschleißrate wurde während des Tests eine elektrische Spannung von 2,5 V zwischen Probenkörper und Ringkörper angelegt. Die angelegte Spannung erzeugt eine Oberflächenladung auf Proben- und Ringkörper, die die Korrosionsbedingungen unter Reinstwasseranwendung des Verbundwerkstoffes simuliert. Als Gleitmittel wird laborübliches vollentsalztes Wasser verwendet. Das während dem Gleitprozess entstehende Abriebvolumen des Probenkörpers wurde zeitabhängig gemessen. Der spezifische Verschleißkoeffizient wird aus der Steigung eines detektierten linearen Verlaufs zwischen Abriebvolumen und Zeit bestimmt.

Gleichzeitig wird der Reibungskoeffizient über das Drehmoment des angetriebenen Ringkörpers ermittelt.

Für vergleichende Untersuchungen an unterschiedlichen Verbundmaterialien werden jeweils die gleichen Verfahrensbedingungen voreingestellt, so dass die ermittelte spezifische Verschleißrate und der Reibungskoeffizient nur von der Zusammensetzung der jeweils untersuchten Verbundmaterialen abhängig sind.

### Herstellung der Verbundmaterialien

### (1) Beispiel 1 - PEEK/CF/GF - Verbundmaterial aus PEEK Carbonfaser und Glasfaser

. Es wird eine Masse von Polyetheretherketon der Firma Victrex, Carbonfasern mit einem Wärmeausdehnungskoeffizienten von -0,1x10⁻⁶/ K und Glasfasern mit einer elektrischen Leitfähigkeit von 1x10⁻⁹ S/m durch Schmelzextrusion zu einem Verbundmaterial kompoundiert und zu einem Probenstück verarbeitet. Carbonfaser und Glasfaser werden zu gleichen Gewichtsanteilen verwendet. Der Anteil der Carbonfasern und der Glasfasern im Probenstück beträgt jeweils 15 Gew.%. Der spezifische Abriebkoeffizient und der Reibungskoeffizient werden über den "Block on the Ring Test" ermittelt. Weiterhin wird die Wärmeausdehnung des Verbundmaterials durch Thermomechanische Analyse bestimmt. Die Werte sind in Tabelle 1 wiedergegeben.

### (2) Beispiel 2, PEEK/CF - Verbundmaterial aus PEEK und Carbonfaser-Vergleichsbeispiel

Es wird eine Masse von Polyetheretherketon der Firma Victrex und Carbonfasern mit einem Wärmeausdehnungskoeffizienten von -0,1x10⁻⁶/ K durch Schmelzextrusion kompoundiert und zu einem Probenstück verarbeitet. Der Anteil der Carbonfasern im Probenstück beträgt 30 Gew.%. Die spezifische Verschleißrate und der Reibkoeffizient werden über den "Block on the Ring Test" ermittelt. Weiterhin wird die Wärmeausdehnung des Verbundmaterials durch Thermomechanische Analyse bestimmt. Die Werte sind in Tabelle 1 wiedergegeben.

### Beispiel 3, PEEK/GF-Verbundmaterial aus PEEK und Glasfaser-Vergleichsbeispiel

Es wird eine Masse von Polyetheretherketon der Firma Victrex, und Glasfasern mit einer elektrischen Leitfähigkeit von 1x10⁻⁹ S/m durch Schmelzextrusion kompoundiert und zu einem Probenstück verarbeitet. Der Anteil der Glasfasern im Probenstück beträgt jeweils 30 Gew.%. Die spezifische Verschleißrate und der Reibungskoeffizient werden über den "Block on the Ring Test" ermittelt. Weiterhin wird die Wärmeausdehnung des Verbundmaterials durch Thermomechanische Analyse bestimmt. Die Werte sind in Tabelle 1 wiedergegeben.

### Beispiel 4 - Vergleichsbeispiel

Es wird ein Probenkörper aus PEEK hergestellt. Die spezifische Verschleißrate und der Reibkoeffizient werden über den "Block on the Ring Test" ermittelt. Weiterhin wird die Wärmeausdehnung des Verbundmaterials bestimmt. Die Werte sind in Tabelle 1 wiedergegeben.

**Tabelle 1**

| **Beispiel** | **Wärmeausdehnungskoeffizie nt [10⁻⁶/K]** | **Reibkoeffizie nt [µs]** | **spezifischer Verschleißrate [10⁻⁶ mm³/Nm]** |
|---|---|---|---|
| (1) PEEK/CF/GF | 7 | 0,14 | 0,9 |
| (2) PEEK/CF | 4 | 0,14 | 9 |
| (3) PEEK/GF | 16 | 0,22 | 8 |
| (4) PEEK | 47 | 0,17 | 4 |

Die Ergebnisse zeigen, dass das erfindungsgemäße Verbundmaterial PEEK/CF/GF einen zum Verbundmaterial PEEK/CF entsprechenden Reibungskoeffizienten aufweist. Dabei ist der Reibungskoeffizient geringer als der des Verbundmaterials PEEK/GF. Der Wärmeausdehnungskoeffizient von PEEK/CF/GF ist geringer als der des Verbundmaterials PEEK/GF, gegenüber Verbundmaterial PEEK/ CF jedoch durch den Anteil an Glasfaser erhöht. Unerwartet ist die spezifische Verschleißrate des Verbundmaterials PEEK/CF/GF deutlich niedriger als die spezifische Verschleißrate der Vergleich Verbundmaterialien PRRK/CF und PEEK/GF.

## Patentansprüche

1. Verbundmaterial für mechanische Bauteile zur Kraft- und Bewegungsübertragung enthaltend oder bestehend aus zumindest drei Komponenten
(i) mindestens 45 Gew.% bezogen auf das Gesamtgewicht des Verbundmaterials eines Trägermaterials bestehend aus Polyetheretherketone,
(ii) 3 bis 20 Gew.% bezogen auf das Gesamtgewicht des Verbundmaterials eines ersten faserförmigen Verstärkungsmaterials, und
(iii) 10 bis 45 Gew.% bezogen auf das Gesamtgewicht des Verbundmaterials eines zweiten Verstärkungsmaterials,
wobei das erste faserförmige Verstärkungsmaterial einen Wärmeausdehnungskoeffizienten aufweist, der geringer ist als der des zweiten Verstärkungsmaterials und wobei das zweite Verstärkungsmaterial eine elektrische Leitfähigkeit aufweist, die geringer ist als die des ersten Verstärkungsmaterials, und wobei sich die Gewichtsanteile der im Verbundmaterial enthaltenen Komponenten auf 100% addieren und
wobei das erste faserförmige Verstärkungsmaterial Carbonfaser enthält oder aus Carbonfaser besteht und
das zweite Verstärkungsmaterial Glasfaser enthält oder aus Glasfaser besteht.

2. Verbundmaterial nach Anspruch 1, **dadurch gekennzeichnet, dass** das erste faserförmige Verstärkungsmaterial einen Wärmeausdehnungskoeffizienten von -0,15x10⁻⁶/K bis 2x10⁻⁶/K parallel zur Faserrichtung aufweist und das zweite Verstärkungsmaterial eine elektrische Leitfähigkeit von 10⁻⁴ S/m oder weniger aufweist.

3. Verbundmaterial nach Anspruch 1 oder 2, **dadurch gekennzeichnet, dass** das erste Verstärkungsmaterial und das zweite Verstärkungsmaterial isotrop in dem Trägermaterial dispergiert sind.

4. Verbundmaterial nach wenigstens einem der vorhergehenden Ansprüche, **dadurch gekennzeichnet, dass** das zweite Verstärkungsmaterial faserförmig ist.

5. Verbundmaterial nach wenigstens einem der vorhergehenden Ansprüche, **dadurch gekennzeichnet, dass** der Anteil des ersten faserförmigen Verstärkungsmaterials in dem Verbundmaterial 3 Gew.% bis 18 Gew.%, bevorzugt 5 Gew.% bis 15 Gew.%, weiter bevorzugt 7 Gew.% bis 15 Gew.%, bezogen auf das Gesamtgewicht des Verbundmaterials, beträgt und oder
**dadurch gekennzeichnet, dass** der Anteil des zweiten Verstärkungsmaterials in dem Verbundmaterial 10 Gew.% bis 40 Gew.%, weiter bevorzugt 15 bis 35%, weiter bevorzugt 15 Gew.% bis 30%, bezogen auf das Gesamtgewicht des Verbundmaterials, beträgt.

6. Verbundmaterial nach wenigstens einem der vorhergehenden Ansprüche, **dadurch gekennzeichnet, dass** der Anteil des Trägermaterials in dem Verbundmaterial mindestens 50 Gew.%, weiter bevorzugt mindestens 55 Gew.%, weiter bevorzugt mindestens 60 Gew.% bezogen auf das Gesamtgewicht des Verbundmaterials beträgt.

7. Verbundmaterial nach wenigstens einem der vorhergehenden Ansprüche, **dadurch gekennzeichnet, dass** das Gewichtsverhältnis des zweiten Verstärkungsmaterials zum faserförmigen ersten Verstärkungsmaterial 3:1 bis 1:1, bevorzugt 2:1 bis 1:1 beträgt.

8. Verbundmaterial nach wenigstens einem der vorhergehenden Ansprüche, **dadurch gekennzeichnet, dass** das Verbundmaterial ein Antioxidationsmittel aufweist,
bevorzug **dadurch gekennzeichnet, dass** es sich bei dem Antioxidationsmittel um ein primäres und/ oder ein sekundäres Antioxidationsmittel handelt.

9. Verbundmaterial nach wenigstens einem der vorhergehenden Ansprüche, **dadurch gekennzeichnet, dass** der Anteil des Antioxidationsmittels in dem Verbundmaterial 0,001 Gew.% bis 2,5 Gew.%, bevorzugt 0,01 Gew.% bis 2,0 Gew.%, weiter bevorzugt 0,1 Gew.% bis 1 Gew.% bezogen auf das Gesamtgewicht des Verbundmaterials, beträgt.

10. Verbundmaterial nach wenigstens einem der vorhergehenden Ansprüche, **dadurch gekennzeichnet, dass** die Fasern des faserförmigen ersten Verstärkungsmaterials und/oder die Fasern des faserförmigen zweiten Verstärkungsmaterials einen Faserdurchmesser von 1 bis 10 µm, bevorzugt 2 µm bis 9 µm, weiter bevorzugt 3 µm bis 8 µm aufweisen und/oder
**dadurch gekennzeichnet, dass** die Fasern des faserförmigen ersten Verstärkungsmaterials und/oder die Fasern des zweiten faserförmigen Verstärkungsmaterials eine Länge 10 µm bis 60 µm, bevorzugt von 15 µm bis 55 µm, weiter bevorzugt von 20 µm bis 50 µm aufweisen.

11. Verbundmaterial nach wenigstens einem der vorhergehenden Ansprüche, **dadurch gekennzeichnet, dass** das Verhältnis des Durchmessers zur Länge der Fasern des ersten faserförmigen Verstärkungsmaterials und der Fasern des zweiten faserförmigen Verstärkungsmaterials größer 1:2 bis 1:20 ist.

12. Verbundmaterial nach wenigstens einem der vorhergehenden Ansprüche, **dadurch gekennzeichnet, dass** die Anteile des Trägermaterials, des ersten faserförmigen Verstärkungsmaterials und des zweitem Verstärkungsmaterials und gegebenenfalls des Antioxidationsmittels zusammen 100 Gew.% ergeben.

13. Mechanisches Bauteil für Kraft- und Bewegungsübertragungen, aufweisend ein Verbundmaterial nach wenigstens einem der Ansprüche 1 bis 12,
wobei es sich bei dem Bauteil bevorzugt um ein Zahnrad handelt.

14. Zahnradpumpe aufweisend mindestens ein Zahnrad nach Anspruch 13.

15. Verwendung eines Verbundmaterials nach wenigstens einem der Ansprüche 1 bis 12 für flüssigkeitsgelagerte mechanische Bauteile in medizinischen Pumpanwendungen,
bevorzugt für flüssigkeitsgelagerte mechanische Bauteile in Dialysepumpen oder Pumpen zur Förderung von Reinstwasser.

## Claims

1. A composite material for mechanical components of force and motion transmission consisting of at least three constituents
(i) at least 45% by weight of a substrate material in relation to the total weight of the composite material consisting of polyether ether ketone,
(ii) 3 to 20% by weight of a first fibrous reinforcing material in relation to the total weight of the composite material, and
(iii) 10 to 45% by weight of a second reinforcing material in relation to the total weight of the composite material, wherein
the first fibrous reinforcing material has a lower thermal expansion coefficient than the second reinforcing material and wherein the second reinforcing material has a lower electrical conductivity than the first reinforcing material and wherein the parts by weight of the constituents contained in the composite material add up to 100%, and
wherein the first fibrous reinforcing material contains or is carbon fiber, and
wherein the second reinforcing material contains or is glass fiber.

2. The composite material according to claim 1, **characterized in that** the first fibrous reinforcing material exhibits a thermal expansion of from -0.15x10⁻⁶/K to 2x10⁻⁶/K parallel to the direction of the fibers and the second reinforcing material has an electrical conductivity of 10⁻⁴ S/m or less.

3. Verbundmaterial nach Anspruch 1 oder 2, **dadurch gekennzeichnet, dass** das erste Verstärkungsmaterial und das zweite Verstärkungsmaterial isotrop in dem Trägermaterial dispergiert sind.

4. The composite material according to at least one of the preceding claims, **characterized in that** the second reinforcing material is fibrous.

5. The composite material according to at least one of the preceding claims, **characterized in that** the amount of the first fibrous reinforcing material in the composite material is 3% to 18% by weight, preferentially 5% to 15% by weight, further preferentially 7% to 15% by weight relative to the total weight of the composite material, and/ or
**characterized in that** the amount of the second reinforcing material in the composite material is to 10% to 40% by weight, further preferentially 15% to 35% by weight, further preferentially 15% to 30% by weight relative to the total weight of the composite material.

6. The composite material according to at least one of the preceding claims, **characterized in that** the amount of the substrate material in the composite material is at least 50% by weight, further preferentially at least 55% by weight, further preferentially at least 60% by weight relative to the total weight of the composite material.

7. The composite material according to at least one of the preceding claims, **characterized in that** the weight ratio of the second reinforcing material to the fibrous first reinforcing material is 3:1 to 1:1, preferentially 2:1 to 1:1.

8. The composite material according to at least one of the preceding claims, **characterized in that** the composite material comprises an antioxidant,
preferentially **characterized in that** the antioxidant is a primary and/or a secondary antioxidant

9. The composite material according to at least one of the preceding claims, **characterized in that** the amount of the antioxidant in the composite material is 0.001% to 2.5% by weight, preferably 0.01% to 2.0% by weight, more preferably 0.1% to 1% by weight, based on the total weight of the composite material.

10. The composite material according to at least one of the preceding claims, **characterized in that** the fibers of the fibrous first reinforcing material and/or the fibers of the fibrous second reinforcing material have a fiber diameter of from 1 µm to 10 µm, preferentially 2 µm to 9 µm, further preferentially 3 µm to 8 µm and/ or
**characterized in that** the fibers of the fibrous first reinforcing material and/or the fibers of the second fibrous reinforcing material have a length of 10 µm to 60 µm, preferentially 15 µm to 55 µm, further preferentially 20 µm to 50 µm.

11. The composite material according to at least one of the preceding claims, **characterized in that** the ratio of diameter to length for the fibers of the first fibrous reinforcing material and the fibers of the second fibrous reinforcing material is greater than 1:2 to 1:20.

12. The composite material according to at least one of the preceding claims, **characterized in that** the amount of the substrate material, the first fibrous reinforcing material and the second reinforcing material and if applicable the antioxidant together add up to 100% by weight.

13. A mechanical component for force and motion transmissions comprising a composite material according to at least one of claims 1 to 12, **characterized in that** the component is a gear.

14. A gear pump comprising at least one gear according to claim 13.

15. Use of a composite material according to at least one of claims 1 to 12 for mechanical components suspended in liquid in medical pumping applications, preferably
for mechanical components suspended in liquid in dialysis pumps or pumps for pumping ultrapure water.

## Revendications

1. Matériau composite pour composants mécaniques pour transmission de force et de mouvement contenant ou constitué d'au moins trois composants
(i) au moins 45 % en poids par rapport au poids total du matériau composite d'un matériau de support constitué de polyéther-éthercétone,
(ii) 3 à 20 % en poids par rapport au poids total du matériau composite d'un premier matériau de renforcement fibreux, et
(iii) 10 à 45 % en poids par rapport au poids total du matériau composite d'un deuxième matériau de renforcement,
dans lequel le premier matériau de renforcement fibreux présente un coefficient de dilatation thermique qui est inférieur à celui du deuxième matériau de renforcement et dans lequel le deuxième matériau de renforcement présente une conductivité électrique qui est inférieure à celle du premier matériau de renforcement, et dans lequel les proportions en poids des composants contenus dans le matériau composite s'additionnent pour un total de 100 % et
dans lequel le premier matériau de renforcement fibreux contient des fibres de carbone ou est constitué de fibres de carbone et
le deuxième matériau de renforcement contient des fibres de verre ou est constitué de fibres de verre.

2. Matériau composite selon la revendication 1, **caractérisé en ce que** le premier matériau de renforcement fibreux présente un coefficient de dilatation thermique de -0,15 x 10⁻⁶/K à 2 x 10⁻⁶/K parallèlement à la direction des fibres et le deuxième matériau de renforcement présente une conductivité électrique de 10⁻⁴ S/m ou moins.

3. Matériau composite selon la revendication 1 ou 2, **caractérisé en ce que** le premier matériau de renforcement et le deuxième matériau de renforcement sont dispersés de manière isotrope dans le matériau de support.

4. Matériau composite selon au moins l'une des revendications précédentes, **caractérisé en ce que** le deuxième matériau de renforcement est fibreux.

5. Matériau composite selon au moins l'une des revendications précédentes, **caractérisé en ce que** la proportion du premier matériau de renforcement fibreux dans le matériau composite atteint 3 % en poids à 18 % en poids, de préférence 5 % en poids à 15 % en poids, encore plus préférablement 7 % en poids à 15 % en poids, par rapport au poids total du matériau composite, et/ou
**caractérisé en ce que** la proportion du deuxième matériau de renforcement dans le matériau composite atteint 10 % en poids à 40 % en poids, plus préférablement 15 à 35 %, encore plus préférablement 15 % en poids à 30 %, par rapport au poids total du matériau composite.

6. Matériau composite selon au moins l'une des revendications précédentes, **caractérisé en ce que** la proportion du matériau de support dans le matériau composite atteint au moins 50 % en poids, plus préférablement au moins 55 % en poids, encore plus préférablement au moins 60 % en poids par rapport au poids total du matériau composite.

7. Matériau composite selon au moins l'une des revendications précédentes, **caractérisé en ce que** le rapport en poids du deuxième matériau de renforcement sur le premier matériau de renforcement fibreux est de 3/1 à 1/1, de préférence 2/1 à 1/1.

8. Matériau composite selon au moins l'une des revendications précédentes, **caractérisé en ce que** le matériau composite présente un agent antioxydant,
de préférence **caractérisé en ce que** l'agent antioxydant est un agent antioxydant primaire et/ou secondaire.

9. Matériau composite selon au moins l'une des revendications précédentes, **caractérisé en ce que** la proportion d'agent antioxydant dans le matériau composite atteint 0,001 % en poids à 2,5 % en poids, de préférence 0,01 % en poids à 2,0 % en poids, plus préférablement 0,1 % en poids à 1 % en poids par rapport au poids total du matériau composite.

10. Matériau composite selon au moins l'une des revendications précédentes, **caractérisé en ce que** les fibres du premier matériau de renforcement fibreux et/ou les fibres du deuxième matériau de renforcement fibreux présentent un diamètre de fibres allant de 1 à 10 µm, de préférence de 2 µm à 9 µm, plus préférablement de 3 µm à 8 µm et/ou
**caractérisé en ce que** les fibres du premier matériau de renforcement fibreux et/ou les fibres du deuxième matériau de renforcement fibreux présentent une longueur allant de 10 µm à 60 µm, de préférence de 15 µm à 55 µm, plus préférablement de 20 µm à 50 pm.

11. Matériau composite selon au moins l'une des revendications précédentes, **caractérisé en ce que** le rapport du diamètre sur la longueur des fibres du premier matériau de renforcement fibreux et des fibres du deuxième matériau de renforcement fibreux est de plus de 1/2 à 1/20.

12. Matériau composite selon au moins l'une des revendications précédentes, **caractérisé en ce que** les proportions du matériau de support, du premier matériau de renforcement fibreux et du deuxième matériau de renforcement et le cas échéant de l'agent antioxydant font conjointement 100 % en poids.

13. Composant mécanique pour des transmissions de force et de mouvement, présentant un matériau composite selon au moins l'une des revendications 1 à 12,
le composant étant de préférence une roue dentée.

14. Pompe à roue dentée présentant au moins une roue dentée selon la revendication 13.

15. Utilisation d'un matériau composite selon au moins l'une des revendications 1 à 12 pour des composants mécaniques placés dans un fluide dans des applications de pompes médicales, de préférence pour des composants mécaniques placés dans un fluide dans des pompes de dialyse ou des pompes pour acheminer l'eau purifiée.
